(19) Europäisches Patentamt / European Patent Office / Office européen des brevets

(11) **EP 2 709 578 B1**

(12) **EUROPEAN PATENT SPECIFICATION**

(45) Date of publication and mention
of the grant of the patent:
**04.01.2017 Bulletin 2017/01**

(51) Int Cl.:
*A61F 13/00* (2006.01)　　*B32B 5/26* (2006.01)
*B32B 5/28* (2006.01)　　*B32B 5/02* (2006.01)
*A61F 13/02* (2006.01)

(21) Application number: **12724035.6**

(22) Date of filing: **17.05.2012**

(86) International application number:
**PCT/US2012/038283**

(87) International publication number:
**WO 2012/158879 (22.11.2012 Gazette 2012/47)**

(54) **TEARABLE ELASTIC COMPOSITE ARTICLES**

ZERREISSBARER ELASTISCHER VERBUNDARTIKEL

ARTICLES COMPOSITES ÉLASTIQUES DÉCHIRABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.05.2011 US 201161487322 P**

(43) Date of publication of application:
**26.03.2014 Bulletin 2014/13**

(73) Proprietor: **3M Innovative Properties Company**
**St. Paul, MN 55133-3427 (US)**

(72) Inventors:
• **FUNG, Simon S.**
**Saint Paul, Minnesota 55133-3427 (US)**
• **BROWN, Mary L.**
**Saint Paul, Minnesota 55133-3427 (US)**
• **ROGERS, John J.**
**Saint Paul, Minnesota 55133-3427 (US)**

(74) Representative: **Vossius & Partner**
**Patentanwälte Rechtsanwälte mbB**
**Siebertstrasse 3**
**81675 München (DE)**

(56) References cited:
**GB-A- 2 200 594**　　**US-A- 4 756 942**
**US-A- 5 209 801**　　**US-A1- 2004 121 683**
**US-A1- 2004 214 494**　　**US-B1- 6 410 464**

**Description**

**BACKGROUND**

Elastic composite articles can be provided, for example, from two coverwebs with elastic strands or filaments located between the coverwebs. When the two coverwebs are attached to each other while the elastic filaments are stretched, the finished composite exhibits elasticity if the elastic filaments are allowed to relax after the coverwebs are attached to each other. Some elastic composite articles such as those described in U.S. Patent Nos. 3,575,782 (Hansen) and 4,984,584 (Hansen et al.) are self-adherent.

Self-adherent elastic composite articles such as elastic wraps, tapes, and bandages are widely utilized in first-aid, sports medicine, and veterinary applications. They can be used, for example, to provide compressive support for injuries or to hold or compresses, medicate pads, and the like in place on a human or an animal. Clinicians, particularly phlebotomists, can utilize self-adherent elastic wraps to hold gauze on limbs after blood draws. The self-adherent or cohesive articles preferably do not stick to skin or a variety of other materials (for example, compresses, medicated pads, and the like), but will cohesively bond to other layers of the article with sufficient force to hold the contacting layers together against reasonably high shearing forces.

To use a self-adherent article, a portion of the desired length may be unrolled or separated from the remainder of the composite remaining on the roll. Separation of the elastic composite article can occur before or after the elastic composite article is applied to an individual. Although the elastic composite articles may exhibit relatively straight tear characteristics in the down-web or machine direction, the elastic composite articles exhibit much less desirable tear characteristics in the cross-web or cross-machine direction.

Attempts to tear (for example, hand tear) the elastic composite articles in the cross-web direction can result in jagged or uneven tear lines that extend over a significant distanced in the down-web direction as well as in the cross-web direction. Such tear properties are undesirable. As a result, users typically resort to scissors or other cutting tools when separating lengths of elastic composite article from a roll. The use of a tool may require the user to let go of the unrolled elastic composite material and/or the roll. Additionally, the use of such tools to cut tape or bandages for multiple patients raises a significant concern of transferring germs or bacteria from one patient to another.

One attempt to provide desirable cross-web tear characteristics in cohesive elastic composite articles involves the use of a warp-knitted fabric oriented with the knit yarns extending longitudinally as described, for example, in U.S. Patent No. 5,762,623 (Murphy et al.). The knitted fabric are, however, relatively expensive. As a result, a cohesive elastic composite article including one or more knitted fabrics is more expensive to manufacture than a comparable elastic composite article using only nonwoven coverwebs. Another potential disadvantage is that the knitted fabric may undesirably increase

the force required to tear the elastic composite article in the cross-web direction and may cause user/patient discomfort due to exposed knit points on the knitted web..

Embossing has also been utilized to form a tear pattern in cohesive elastic composite articles in order to facilitate better tearing, as described, for example, in U.S. Patent No. 7,135,213 (Maki et al.). But, this method of improving tearability also has disadvantages. For example, special embossing equipment is required as part of the process for making the nonwoven coverwebs; otherwise, an additional separate embossing step is required, which increases manufacturing costs.

**SUMMARY**

In view of the foregoing, we recognize that there is a need in the art for self-adherent elastic composite articles that exhibit improved cross-web tear characteristics and that can be simply and inexpensively manufactured.

The present invention is defined by the features of the claims.

Briefly, in one aspect, the present provides an elastic composite article comprising a nonwoven fibrous coverweb, a woven scrim attached to the coverweb, and a plurality of spaced elastic yarns located between the coverweb and the woven scrim. The elastic composite article is uniformly impregnated throughout and bonded together in a unified structure with a polymeric binder. The woven scrim has a filament mass density (1 denier = 1 gram per 9000 meters) of at least about 40 denier in the machine direction and at least about 70 denier in the cross-web direction. In some preferred embodiments, the woven scrim has a filament mass density of at least about 40 denier in the machine direction and at least about 150 denier in the cross-web direction.

The elastic composite articles of the invention exhibit improved cross-web tear characteristics. In addition, the elastic composite articles of the invention are softer/less stiff than elastic composite materials including knitted fabrics. In certain implementations, the composite articles have a smoother finish and/or feel less scratchy than a knitted counterpart.

The terms "comprises" and variations thereof do not have a limiting meaning where these terms appear in the description and claims.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As recited herein, all numbers should be considered modified by the term "about".

As used herein, "a," "an," "the," "at least one," and "one or more" are used interchangeably. Thus, for example, a system comprising "a" nonwoven fibrous web can be interpreted as a system comprising "one or more" nonwoven fibrous webs.

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments. In several places throughout the application, guidance is provided through lists of examples, which examples can be used in various combinations. In each instance, the recited list serves only as a representative group and should not be interpreted as an exhaustive list.

## BRIEF DESCRIPTION OF THE DRAWINGS

The invention will be further described with reference to the drawings, wherein corresponding reference characters indicate corresponding parts throughout the several views, and wherein:

FIG. 1 is a schematic representation of an elastic composite article according to an aspect of the present disclosure.
FIG. 2 is a schematic representation showing the manufacture of an elastic composite article.
FIG. 3 is a representation in plan view of a portion of an elastic composite article

Layers in certain depicted embodiments are for illustrative purposes only and are not intended to absolutely define the thickness, relative or otherwise, location, or specific orientation of any component. While the above-identified figures set forth several embodiments of the invention, other embodiments are also contemplated, as noted in the discussion. In all cases, this disclosure presents the invention by way of representation and not limitation. It should be understood that numerous other modifications and embodiments can be devised by those skilled in the art, which fall within the scope and spirit of the principles of the invention.

## DETAILED DESCRIPTION OF ILLUSTRATIVE EMBODIMENTS

The tearable elastic composite articles of the invention are made of a self-adhering material (for example, a self-adhering elastomeric material), which is capable of exerting a compressive force when extended. The composite articles include a nonwoven fibrous web, a woven scrim, a plurality of generally longitudinally extending elastic yarns. The composite article is typically coated or impregnated with a polymeric binder. In some embodiments, the elastic composite article comprises a plurality of generally longitudinally extending, partially extended or non-extended elastic yarns bound with a polymeric binder between the nonwoven web and the woven scrim. Preferably, the polymeric binder is cohesive so that the elastic substrate is self-adherent, but will not adhere to clothing, hair, skin, or the like. Suitable polymeric binders providing cohesive properties may be either elastomeric or non-elastomeric polymeric binders; however, preferably, the polymeric binder is an elastomeric polymeric binder due to the generally favorable properties of such binders such as long-term flexibility, extensibility, and/or elasticity. Suitable elastomeric polymeric binders may comprise natural rubber latex, a synthetic latex such as homopolymer and copolymer latexes of acrylics, butadienes, styrene/butadiene rubbers, chloroprenes, ethylenes (for example, vinyl acetate/ethylene), isoprenes, nitriles and urethanes, or mixtures thereof. Examples of suitable polymeric elastomeric binders are disclosed, for example, in U.S. Patent Nos. 3,575,782; 4,984,585; and 6,156,424 as well as in textbooks such as Neoprene Latex: Principles of Compounding and Processing, J.C. Carl, 1962, Delaware, E. I. DuPont de Nemours (for example, under the section entitled Contact Bond Adhesives on page 100) and Handbook of Adhesives 3rd Edition, Skeist, 1990, New York, Van Nostrand Reinhold (for example, page 305).

For configurations including elastic yarns bound between the nonwoven web and the woven scrim, partially extended yarns are preferred. During manufacturing of the elastic composite material (for example, during binding of elastic yarns with polymeric binder between the nonwoven web and the woven scrim), it is preferable to stretch the yarns to a length of 5 times or less (more preferably, 3.5 times of less) times their fully relaxed length. Generally, a draw ratio of about 2.5:1 to about 3.5:1 is desirable. Preferably, the ends per inch (epi) is less than 15; more preferably, 12 or less; most preferably, 10 epi or less. Within this range, an epi of 4 or more is suitable; 5 or more is more suitable; 6 or more is most suitable. Preferably, elastic yarns have a denier less than 550; more preferably, about 350 or less. Within this range, a denier of 100 or more is suitable; 150 or more is more suitable; and 200 or more is most suitable.

Nonwoven webs can be formed using a variety of processes such as melt blowing, spun bonding processes, carding, needle punched web making processes, air laid web making processes, wet laid web making processes, film aperturing processes, stable fiber carding processes, hydroentanglement, and the like. Hydroentanglement is described, for example, in U.S. Patent Nos. 3,485,706; 3,486,168; 3,493,462; 3,494,821; and 3,508,228. Another method of forming a nonwoven web is needle-tacking as described, for example, in U. S. Patent No. 5,016,331. Other suitable webs includes CEREX nylon spunbond nonwovens, available from Cerex, Cantonment, FL.

In general, when two layers of nonwoven materials are layered together, the final appearance may appear to be more uniform since the lack of coverage in either layer is usually not overlapping. Since the composite articles of the present disclosure include a woven scrim, the final product might appear to be non-uniform or comprise an increased number of "holes", and may accordingly provide an objectionable appearance to some users. The uniformity may be improved by increasing the total weight of the nonwoven layer.

The total weight of nonwoven webs used in the elastic composite articles of the invention can be, for example, 50 grams per square meter (gsm) or less per web. Further limits on the total weight of the web may be beneficial. For example, the webs may have a total weight per web of 20 gsm or less, even 15 gsm or less, even 13 or less, and even 10 gsm or less. In certain implementations, a total weight of 10 gsm provides sufficient uniformity balanced against desired hand tearable characteristics.

Woven scrims are included in the elastic composite articles of the invention to improve their cross-web hand-tear characteristics. Woven scrims useful in the present invention have a filament mass density of at least about 40 denier in the machine direction (MD) and at least about 70 denier in the cross-web direction (CD). In some preferred embodiments, the woven scrim has a filament mass density of at least about 40 denier in MD and at least about 150 denier in CD. The use of finer denier filaments (e.g., 40 denier) can have a substantial impact on the ease of tear across the bandage. Typically, the ends per inch (epi) in MD is no greater 25; in certain aspects no greater than 20; in certain aspects no greater than 16, in certain aspects no greater than 12. Typically, the ends per inch (epi) in CD is no greater than 15; in certain aspects no greater than 12; in certain aspects no greater than 10; in certain aspects no greater than 8, in certain aspects no greater than 6. Particular suitable MD to CD pairings include 16 x 8, 18 x 8, 20 x 8, but may vary depending on the filament mass density (i.e., denier) in one or both directions. In some embodiments, the woven scrim comprises polyester, cotton, or blends thereof. Suitable woven scrims are commercially available from Milliken & Company (Spartanburg, SC).

Woven scrims of the present disclosure typically include a first tensile strength (as measured in the Examples below) in the machine direction and a second tensile strength in the cross-web direction. In certain implementations, it can be preferred that the second, cross-web tensile strength is significantly greater than the first, machine direction tensile strength such that the ratio of first to second (MD/CD) tensile strengths is no greater than 0.8. In other implementations, the MD/CD ratio is no greater than 0.7, in some embodiments, no greater than 0.6 and in yet other embodiments no greater than 0.5. When the tensile strength in the CD direction is higher than the MD, the propensity of tearing down web during the action of tearing across is reduced.

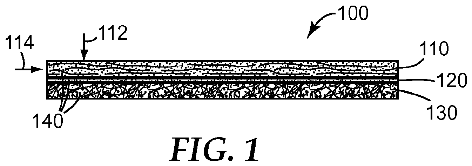

An exemplary composite article 100 is depicted in FIG. 1 and includes a plurality of elastic yarns 120 disposed on a nonwoven fibrous web 130. The plurality of elastic yarns 120 can be coextensive with the nonwoven web 130. A woven scrim 110 is disposed on the plurality of elastic yarns 120. The composite article 100 includes a cross-web direction 112 and a machine (i.e., unwind or down web) direction 114. The elastic yarns 120 are bound by a polymeric binder 140 between the nonwoven coverweb 130 and the woven scrim 110. As described above, the polymeric binder 140 is preferably impregnated throughout all layers of the composite article 100. It should be appreciated that the orientation of article 100 may be reversed, such that nonwoven coverweb 130 is disposed on top and the woven scrim is diposed on the base. The elastic composite article 100 using one or more of the materials discussed above can exhibit improved tear characteristics in cross-web direction 112, while reducing or preventing unwanted tears in the unwind direction 114.

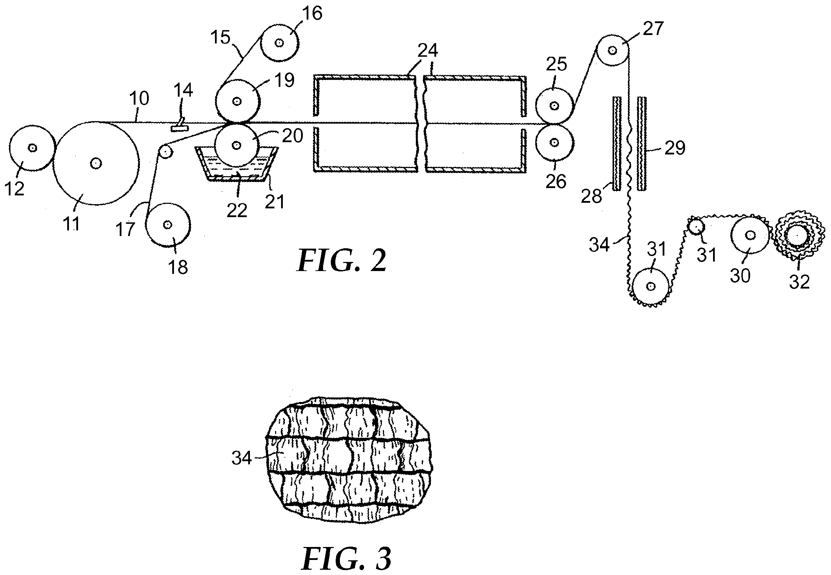

Elastic composite articles of the invention can be made essentially as described in U.S. Patent No. 4,984,584 (Hansen et al.) using equipment as shown in FIG. 2. Elastic yarns 10 from a beam 11 are unwound under tension controlled by driven press roll 12 and through comb 14. A woven scrim 15 along with a thin non-woven fibrous web 17, from supply drums 16 and 18, respectively, or directly from the forming machine, if desired, are brought into contact with the yarns and with each other between rubber-covered squeeze roll 19 and knurled steel squeeze roll 20, the latter dipping into a pan 21 containing a fluid binder mixture 22 and depositing the binder mixture throughout the web 17. The composite web passes directly into a drying oven 24 and thence between pull drums 25 and 26. The web next passes around roll 27, between heating platens 28 and 29, around idler rolls 31 and surface winder roll 30, and is wound up to form stock roll 32.

Squeeze rolls 19 and 20 rotate at a considerably greater surface speed than beam 11, and the yams 10 are accordingly stretched a corresponding amount. This stretch is maintained by operating pull drums 25 and 26 and turn-around drum 27 at approximately the same speed compared with rollers 19 and 20. Surface winder roll 30 and wind-up drum 32, however, are again operated at a slower speed to permit shrinkage of the web as it passes between the heater platens 28 and 29. The composite web 34, which is smooth as it reaches the roll 27, becomes increasingly puckered or shirred as it passes through the heating zone, the result being further indicated in FIG. 3.

The heat supplied by the platens 28 and 29 is sufficient to cause considerable fuming of the sheet material and to relax the structure sufficiently to permit the elastomeric yarns to retract and produce the desired degree of puckering or shirring as controlled by the speed of the surface winder roll. The temperature may be regulated by adjusting both the energy input to the platens and the distance between the platens and the web. In a typical installation for producing a finished web, the electrically heated platens are each 48 inches high, and are spaced between six and nine inches from the web. The platens are operated 600°F at idle and 525° F when the web is running. The duration of the heat treatment may be regulated, for a given length of platen, by adjusting the speed of travel of the web, sufficient time being provided to permit retraction of the web to the desired degree. The platens are maintained at a temperature sufficient to keep the web taut during the shrinking operation between rolls 27 and 30 at the speed indicated but not so high as to cause deterioration of the web as evidenced by excessive fuming and discoloration thereof. The length of the relaxed web after retraction will be within the range of about one-third to about two-thirds the fully extended length. The elastic yarns are initially stretched to a length of about three to five times (and preferably three to three and one-half times) their fully relaxed length (the ratio of stretched length to relaxed length of the yarns is referred to as the draw ratio), and are permitted to relax only partially during the puckering step. Nevertheless, the shirred product is dimensionally stable, the heat treatment serving to provide an effective degree of heat-setting or stabilizing, and neither shrinks nor expands when allowed to stand at normal temperatures and under no external stress; and it returns to such dimensions when first stretched and then permitted to retract.

The thin fibrous mat is conveniently prepared on a carding machine or on a "Rando-Webber" machine. Mats of polyester or rayon staple fibers or mixtures are preferred. The fibers are desirably of about 1.75 denier and about 1.5 inch in length, and the mat is about 0.25 to 0.50 oz./sq. yd. or about 5-10 lb. per 320 sq. yd. These very thin mats are fragile and flimsy, but show surprising strength when combined in composite structures of the type and in the manner hereinabove indicated. The mat as first formed is preferably reinforced by lightly treating it with a compatible bonding agent. As an example, the reinforced mat may consist of 75 parts by weight of polyester staple fibers and 25 parts of polyethyl acrylate, the latter being applied at the forming machine by saturating with a dilute emulsion of the polymer, removing the excess between squeeze rolls and drying in an oven.

Concentrated natural rubber latex or synthetic rubber latex is preferred as the impregnating and bonding or unifying medium. Other elastomers or blends of elastomers having similar properties may be used. The dried rubbery residue, although presenting a slightly tacky feel, does not adhere to the skin, but cohesively bonds to itself with sufficient force to hold the contacting layers together against reasonably high shearing stresses. The impregnating and bonding materials may be used without further modification, but will ordinarily be blended with pigments or other visual modifiers.

## EXAMPLES

Objects and advantages of this invention are further illustrated by the following examples, but the particular materials and amounts thereof recited in these examples, as well as other conditions and details, should not be construed to unduly limit this invention.

### Examples 1 - 10

Elastic composite articles of the invention were prepared essentially as described in U.S. Patent No. 7,854,716 (Schuren et al.) at column 15, lines 18 - 30, with the following exceptions:

- The Spandex yarns used were 210 denier at 10 epi.
- The draw ratio was between 2.5:1 and 3.5:1.
- One of the nonwoven webs in each composite was replaced with a polyester woven scrim from Milliken & Company.
- The resulting sheet material was slit into strips 3 inches wide and 5 yards long (stretched dimensions).

The particular composition of the composite articles, as well as characteristics of the particular woven scrims are shown in Table 1 below. Characteristics of examples 1 - 10 and comparative commercially available samples (comparative examples C-1 - C-5) were measured as described below. The results are shown in Table 2.

**Table 1**

| | Construction | Nonwoven web total weight (gsm) | Woven Fiber denier (MD) | Woven Fiber denier (CD) | Woven epi (MD) | Woven epi (CD) |
|---|---|---|---|---|---|---|
| C-1 3M Coban | nonwoven/ elastic filament / nonwoven | - | - | - | - | - |
| C-2 Renfew Renflex ET | embossed nonwoven / filament | - | - | - | - | - |
| C-3 Andover Coflex NL | knit / elastic filament / nonwoven | - | - | - | - | - |
| C-4 Medico Pro-Trainer | knit elastic filament | - | - | - | - | - |
| C-5 Andover Powerflex | knit with elastic filaments | - | - | - | - | - |
| Example 1 | woven scrim / elastic filament / nonwoven | 10 | 40 | 70 | 20 | 7 |
| Example 2 | woven scrim / elastic filament / nonwoven | 10 | 40 | 70 | 20 | 10 |
| Example 3 | woven scrim / elastic filament / nonwoven | 10 | 40 | 150 | 20 | 8 |
| Example 4 | woven scrim / elastic filament / nonwoven | 10 | 40 | 150 | 16 | 8 |
| Example 5 | woven scrim / elastic filament / nonwoven | 12.5 | 40 | 150 | 16 | 8 |
| Example 6 | woven scrim / elastic filament / nonwoven | 15 | 40 | 150 | 16 | 8 |
| Example 7 | woven scrim / elastic filament / nonwoven | 10 | 40 | 150 | 18 | 8 |
| Example 8 | woven scrim / elastic filament / nonwoven | 12.5 | 40 | 150 | 18 | 8 |
| Example 9 | woven scrim / elastic filament / nonwoven | 10 | 40 | 150 | 20 | 8 |
| Example 10 | woven scrim / elastic filament / nonwoven | 12.5 | 40 | 150 | 20 | 8 |

**Tensile and Elongation Test**

Tensile strength at break and elongation at break in the machine direction (MD) and cross direction (CD) were determined using a Constant Rate of Extension/Tensile Tester (available from Instron or Zwick) essentially according to ASTM D882.

**Tear Test**

<u>Resistance</u>

The resistance of the samples to tearing in the cross direction was determined using an Elmendorf Tear Tester, Model 1600.
The Elmendorf Test Machine is available from Testing Machines Inc. (New Castle, Delaware). Samples with 3 inches width and 4 inches in length are used. In this case, only one layer of fabric is tested and the edge of the test roll is lined up against the cutting blade. The specimen is securely mounted in the jaws and the bottom edge rests evenly on the bottom of the two jaws. There is at least one inch or 20 mm of materials clamped in each jaw with equal amounts of overlap on either end. The initial slit is done by pressing the handle of the knife blade. With the pointer resting against its stop, the lever is depressed such that the pendulum swings to cut. The pendulum is stopped on the return swing without disturbing the position of the pointer.

The reading is obtained from the scale and converted based on the following formula:

$$\text{Grams per ply} = 16 \times \text{Result obtained in scale for one layer}$$

Hand Tear

Samples with 3 inches in width and 4 inches in length were torn by hand in the cross-web direction. The relative ease of tearing the sample was adjudged on a four point scale. Samples were also evaluated for undesired tearing or run in the machine direction (i.e., down web) during the cross-web tear.

1. Very tough to initiate tear, high tear force, or will tear down web
2. Tough to initiate tear and sometimes the tear will go down web
3. Good to tear and does not tear down web most of the time
4. Easy to tear cross-web without going down web

**Table 2**

| | Tensile MD (kg/m) | Tensile MD (kg/m) | Tensile MD/CD | Tear CD (g/ply) | Hand Tear Rating in CD 1-4 (1=poor, 4=good) | MD run during tear |
|---|---|---|---|---|---|---|
| C-1 | 246.4 | 146.4 | 1.7 | 592 | 1 | Yes |
| C-2 | 132.1 | 226.8 | 0.6 | 448 | 3 | Yes |
| C-3 | 264.3 | 366.1 | 0.7 | 560 | 3 | No |
| C-4 | 275.0 | 355.4 | 0.8 | 672 | 4 | No |
| C-5 | 414.3 | 662.5 | 0.6 | 1056 | 4 | No |
| Example 1 | 275.0 | 253.6 | 1.1 | 608 | 2 | Yes |
| Example 2 | 255.4 | 326.8 | 0.8 | 560 | 3 | Yes |
| Example 3 | 233.2 | 459.3 | 0.5 | 504 | 4 | No |
| Example 4 | 217.9 | 441.1 | 0.5 | 1171 | 4 | No |
| Example 5 | 212.5 | 439.3 | 0.5 | 1304 | 4 | No |
| Example 6 | 235.7 | 353.6 | 0.7 | 1374 | 3 | No |
| Example 7 | 214.3 | 292.9 | 0.7 | 1048 | 4 | No |
| Example 8 | 225.0 | 346.4 | 0.6 | 1085 | 3 | No |
| Example 9 | 239.3 | 316.1 | 0.8 | 1064 | 4 | No |

(continued)

|  | Tensile MD (kg/m) | Tensile MD (kg/m) | Tensile MD/CD | Tear CD (g/ply) | Hand Tear Rating in CD 1-4 (1=poor, 4=good) | MD run during tear |
|---|---|---|---|---|---|---|
| Example 10 | 230.4 | 337.5 | 0.7 | 1254 | 3 | No |

As shown in Table 1, when a woven scrim with a fabric diameter machine direction/cross-web direction ratio of close to about 0.5 was incorporated into the elastic composite, it had very good straight tear characteristics.

It should be understood that this invention is not intended to be unduly limited by the illustrative embodiments and examples set forth herein and that such examples and embodiments are presented by way of example only with the scope of the invention intended to be limited only by the claims set forth herein as follows.

**Claims**

1. An elastic composite article comprising:

    a nonwoven fibrous coverweb;
    a woven scrim coupled to the coverweb; and
    a plurality of spaced elastic yarns disposed between the coverweb and the woven scrim

    wherein the elastic composite article is bonded together in a unified structure with a polymeric binder, and wherein the article is self-adherent.

2. The article of claim 1, wherein the polymeric binder is uniformly impregnated in the coverweb, the woven scrim, and the plurality of spaced elastic yarns.

3. The article of claim 1-2, wherein the woven scrim has a filament mass density of at least 40 denier in the machine direction and at least 70 denier in the cross-web direction.

4. The article of claims 1-3, wherein the woven scrim has an ends per inch of no greater than 25 in the machine direction and no greater than 15 in the cross-web direction.

5. The article of claim 1-4, wherein the woven scrim includes a first tensile strength in the machine direction and a second tensile strength in the cross-web direction, wherein the ratio of first to second tensile strengths is no greater than 0.8.

6. The article of claim 5, wherein the ratio of first to second tensile strength and in yet other embodiments no greater than 0.5.

7. The article of any of the preceding claims, wherein the nonwoven web has a total weight of no greater than 15 gsm.

8. The article of any of the preceding claims, wherein the elastomeric polymeric binders is selected from the group consisting of a natural rubber latex, a synthetic latex, and combinations thereof.

9. The article of any of the previous claims, wherein the plurality of elastic yarns comprise partially extended yarns.

10. The article of any of the previous claims, wherein plurality of elastic yarns comprise an ends per inch of at least 4 and no greater than 15.

11. The article of any of the previous claims, wherein the plurality of elastic yarns have a denier of at least 100 and no greater than 550.

12. The article of any of the previous claims, wherein the article does not adhere to clothing, hair or skin.

**Patentansprüche**

1. Elastischer Verbundstoffartikel, umfassend:

eine Vliesfaserdeckbahn;
ein Gewebegelege, das mit der Deckbahn verbunden ist; und
mehrere beabstandete, elastische Fäden, die zwischen der Deckbahn und dem Gewebegelege angeordnet sind,

wobei der elastische Verbundstoffartikel in einer einheitlichen Struktur mit einem polymeren Bindemittel zusammengefügt ist, und
wobei der Artikel selbsthaftend ist.

2. Artikel nach Anspruch 1, wobei das polymere Bindemittel gleichmäßig in die Deckbahn, das Gewebegelege und die mehreren beabstandeten, elastischen Fäden imprägniert ist.

3. Artikel nach den Ansprüchen 1 bis 2, wobei das Gewebegelege eine Filamentmassendichte von mindestens 40 Denier in der Maschinenlaufrichtung und mindestens 70 Denier in der Querbahnrichtung aufweist.

4. Artikel nach den Ansprüchen 1 bis 3, wobei das Gewebegelege eine Kettfädenzahl pro Zoll von nicht mehr als 25 in der Maschinenlaufrichtung und nicht mehr als 15 in der Querbahnrichtung aufweist.

5. Artikel nach den Ansprüchen 1 bis 4, wobei das Gewebegelege eine erste Zugfestigkeit in der Maschinenlaufrichtung und eine zweite Zugfestigkeit in der Querbahnrichtung aufweist, wobei das Verhältnis von erster zu zweiter Zugfestigkeit nicht größer als 0,8 ist.

6. Artikel nach Anspruch 5, wobei das Verhältnis von erster zu zweiter Zugfestigkeit nicht größer als 0,5 ist.

7. Artikel nach einem der vorstehenden Ansprüche, wobei die Vliesbahn ein Gesamtgewicht von nicht mehr als 15 Gramm pro Quadratmeter besitzt.

8. Artikel nach einem der vorstehenden Ansprüche, wobei das elastomere, polymere Bindemittel ausgewählt ist aus der Gruppe bestehend aus einem Natur-kautschuklatex, einem Syntheselatex und Kombinationen davon.

9. Artikel nach einem der vorstehenden Ansprüche, wobei die mehreren elastischen Fäden teilweise gestreckte Fäden umfassen.

10. Artikel nach einem der vorstehenden Ansprüche, wobei die mehreren elastischen Fäden eine Kettfädenzahl pro Zoll von mindestens 4 und nicht mehr als 15 umfassen.

11. Artikel nach einem der vorstehenden Ansprüche, wobei die Mehrzahl von elastischen Fäden einen Denier-Wert von mindestens 100 und nicht mehr als 550 aufweist.

12. Artikel nach einem der vorstehenden Ansprüche, wobei der Artikel nicht an Kleidung, Haar oder Haut haftet.

**Revendications**

1. Article composite élastique comprenant :

une bande de recouvrement fibreuse non tissée ;
un canevas tissé couplé à la bande de recouvrement ; et
une pluralité de fils élastiques espacés disposés entre la bande de recouvrement et le canevas tissé

dans lequel l'article composite élastique est lié en une structure unifiée avec un liant polymère et
dans lequel l'article est auto-collant.

2. Article de la revendication 1, dans lequel le liant polymère est imprégné uniformément dans la bande de recouvrement, le canevas tissé et la pluralité de fils élastiques espacés.

3. Article des revendications 1 à 2, dans lequel le canevas tissé a une densité de masse de filament d'au moins 40 deniers dans la direction machine et d'au moins 70 deniers dans la direction transversale de la bande.

4. Article des revendications 1 à 3, dans lequel le canevas tissé a un taux de bouts par pouce non supérieurs à 25 dans la direction machine et non supérieur à 15 dans la direction transversale de la bande.

5. Article des revendications 1 à 4, dans lequel le canevas tissé comprend une première résistance à la traction dans la direction machine et une seconde résistance à la traction dans la direction transversale de la bande, dans lequel le rapport de la première à la seconde résistance à la traction n'est pas supérieur à 0,8.

6. Article de la revendication 5, dans lequel le rapport de la première à la seconde résistance à la traction n'est pas supérieur à 0,5.

7. Article de l'une quelconque des revendications précédentes, dans lequel la bande non tissée a un poids total non supérieur à 15 grammes par m$^2$.

8. Article de l'une quelconque des revendications précédentes, dans lequel les liants polymères élastomères sont choisis dans le groupe constitué d'un latex de caoutchouc naturel, d'un latex synthétique et de leurs combinaisons.

9. Article de l'une quelconque des revendications précédentes, dans lequel la pluralité de fils élastiques comprend des fils partiellement étendus.

10. Article de l'une quelconque des revendications précédentes, dans lequel la pluralité de fils élastiques comprend un taux de bouts par pouce d'au moins 4 et non supérieur à 15.

11. Article de l'une quelconque des revendications précédentes, dans lequel la pluralité de fils élastiques a un denier d'au moins 100 et non supérieur à 550.

12. Article de l'une quelconque des revendications précédentes, dans lequel l'article ne colle pas aux vêtements, aux cheveux ni à la peau.

FIG. 1

*FIG. 2*

*FIG. 3*

EP 2 709 578 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3575782 A, Hansen
- US 4984584 A, Hansen
- US 5762623 A, Murphy
- US 7135213 B, Maki
- US 4984585 A
- US 6156424 A
- US 3485706 A
- US 3486168 A
- US 3493462 A
- US 3494821 A
- US 3508228 A
- US 5016331 A
- US 7854716 B, Schuren

**Non-patent literature cited in the description**

- Contact Bond Adhesives. **J.C. CARL.** Neoprene Latex: Principles of Compounding and Processing. 1962, 100
- Handbook of Adhesives 3rd Edition, Skeist. Van Nostrand Reinhold, 1990, 305